# EUROPÄISCHE PATENTANMELDUNG

(11) **EP 1 887 015 A2**
(43) Veröffentlichungstag der Anmeldung: **13.02.2008**
(21) Anmeldenummer: 07116049.3
(22) Anmeldetag: 11.07.2001
(51) Int. Cl.: C07K 14/47, A61K 38/17, C12N 15/12

(54) **Verfahren zur Gewinnung und Anwendung neuer humaner Defensine als biologisch aktive Eiweißstoffe zur Behandlung von Infektionen und anderen Erkrankungen**

(30) Priorität: 11.07.2000 DE 10033505
(62) Teilanmeldung aus: 01969365.4
(71) Anmelder: IPF Pharmaceuticals GmbH, 30625 Hannover (DE)
(72) Erfinder: Forssmann, Wolf-Georg, Prof. Dr. Dr., 30175 Hannover (DE); Conejo-Garcia, Jose-Ramon, Dr., 30625 Hannover (DE); Adermann, Knut, Dr., 30625 Hannover (DE)
(74) Vertreter: Meyers, Hans-Wilhelm

(57) **Zusammenfassung**

Die Erfindung betrifft die neue Peptide aus menschlichem Blut, hBD-5 (humanes beta-Defensin-5), , hBD-7, hBD-8, hBD-10, hBD-11, hBD-12, hBD-13, hBD-14, hBD-15, hBD-16, hBD-17, hBD-18, hBD-19, hBD-20, hBD-22, hBD-23, hBD-24, hBD-25, hBD-26, hBD-27, hBD-28, hBD-29, hBD-30, hBD-31 und hBD-32 und ihre Derivate, die zum Zwecke der therapeutischen, diagnostischen und gewerblichen Verwendung als Arzneimittel in ihrer Struktur aufgeklärt wurde. Die Peptide können mittels biotechnologischer, rekombinanter Verfahren, chemischer Synthese sowie aus korrespondierenden Vorläuferproteinen proteolytisch hergestellt werden.

## Beschreibung

Die Erfindung betrifft Peptide vom human Defensintyp, ein Verfahren zur Gewinnung von dieser Peptide in reiner oder partiell aufgereinigter Form aus menschlichen und tierischen Körperflüssigkeiten, die die Fähigkeit besitzten, die bakterielle Invasion bei Entzündungserkrankungen zu verhindern, Nukleinsäuren, die für diese Peptide kodieren, Arzneimittel enthaltend diese Peptide, sowie Verwendungen dieser Peptide zur Behandlung verschiedener Erkrankungen.

Diese Peptide lassen sich insbesondere aus Haemofiltrat oder Haemodialysat aus menschlichem und tierischem Blut gewinnen. Diese Stoffe sind als humane Defensine klassifiziert und können zum Zwecke (1) der medizinischen und gewerblichen Verwendung als Medikament und (2) der Analyse von Erkrankungen benutzt werden.

Die Stoffe mit den Kurzbezeichnungen hBD-5 (humanes beta-Defensin-5), hBD-6, hBD-7, hBD-8, hBD-10, hBD-11, hBD-12, hBD-13, hBD-14, hBD-15, hBD-16, hBD-17, hBD-18, hBD-19, hBD-20, hBD-22, hBD-23, hBD-24, hBD-25, hBD-26, hBD-27, hBD-28, hBD-29, hBD-30, hBD-31 und hBD-32, wurden erstmals aus dem Haemofiltrat Nierenkranker nach Ultrafiltration am Haemodialyseapparat gewonnen und über einen antibakteriellen Hemmtest funktionell charakterisiert. Zur Darstellung der Defensinpeptide wurde ein patentiertes Verfahren (Forssmann, 1988; DE 3633707 C1) verfeinert, welches zuvor für Gewinnung von Eiweißstoffen aus Haemofiltrat erfunden wurde. Aus den mit diesem Verfahren gewonnenen Molekülen mit einem Molekulargewicht unter 20 kDalton, die bei veno-venöser oder arterio-venöser Shuntverbindung abfiltriert werden, können die Peptidfraktionen enthaltend die humanen Defensinpeptide durch einen Funktionstest erkannt werden. Das bisher bekannte Verfahren wurde benutzt, um die Rohpeptidextrakte zu gewinnen, mit denen bei der Anwendung des LEHRERschen Radialdiffusionstest ein starker Effekt festgestellt wurde, indem das Wachstum von Bakterien in Kultur unter dem Einfluss dieser Substanz stark gehemmt wird.

Es wurde weiter festgestellt, dass bei weiteren Reinigungsverfahren diese biologischen Aktivitäten konzentriert werden konnten, bis schließlich verschiedene einheitliche Eiweißstoffe identifiziert und in ihrer Struktur aufgeklärt wurden. Vorteilhafterweise können diese Stoffe aus dem bisher als wertlos betrachteten Haemofiltrat aufgereinigt werden, um als wirtschaftlich verwertbare Substanzen benutzt zu werden. Die erfindungsgemäßen Peptide lassen sich durch chemische Synthese und durch gentechnologische Produktion gewinnen, sie lassen sich einsetzen u.a. als pathognomonisches Diagnosemerkmal für die Analyse von entzündlichen Erkrankungen des Magen-Darm-, Respirations- und Urogenitaltraktes sowie anderer Epithelorgane.

Die vorliegende Erfindung betrifft Peptide mit der Aminosäuresequenz Z_{N}-C-Xₘ-X₁-X-C-X₂-Xₙ-C-X-X-X-X₃-Xₒ-C-Xₚ-C-C-Z_{c}
wobei Z_{N} ein Aminosäurerest oder ein Peptidrest von bis zu 30 Aminosäuren , Z_{C} ein Aminosäurerest oder ein Peptidrest von bis zu 30 Aminosäuren,
X = eine beliebige Aminosäure
Xₘ = 3-6 beliebige Aminosäuren
Xₙ = 2-3 Aminosäuren
Xₒ = 5-9 Aminosäuren
Xₚ = 4-6 Aminosäuren
X₁= G, A oder P
X₂= R, K, W, Q oder A ist
X₃= E oder H ist.

Insbesondere bevorzugt werden Peptide mit den folgenden Sequenzen:
(a) hBD-5
   Z_{N2}-CRVRGGRCAVLSCLPKEEQIGKCSTRGRKCC-Z_{C2}
(b) hBD-6
   Z_{N3}-CGYGTARCRKKCRSQEYRIGRCPNTYACC-Z_{C3}
(c) hBD-7
   Z_{N4}-CRRSEGFCQEYCNYMETQVGYCSKKKDACC-Z_{C4}
(d) hBD-8
   Z_{N5}-CKLGRGKCRKECLENEKPDGNCRLNFLCC-Z_{C5}
(e) hBD-10
   Z_{N7}-CHMQQGICRLFFCHSGEKKRGICSDPWNRCC-Z_{C7}
(f) hBD-11
   Z_{N8}-CERPNGSCRDFCLETEIHVGRCLNSRPCC-Z_{C8}
(g) hBD-12
   Z_{N9}-CNKLKGTCKNNCGKNEELIALCQKSLKCC-Z_{C9}
(h) hBD-13
   Z_{N10}-CLNLSGVCRRDVCKVVEDQIGACRRRMKCC-Z_{C10}
(i) hBD-14
   Z_{N11}-CWGKSGRCRTTCKESEVYYILCKTEAKCC-Z_{C11}
(j) hBD-15
   Z_{N12}-CWNFRGSCRDECLKNERVYVFCVSGKLCC-Z_{C12}
(k) hBD-16
   Z_{N13}-CWNNYVQGHCRKICRVNEVPEALCENGRYCC-Z_{C13}
(l) hBD-17
   Z_{N14}-CWNLYGKCRYRCSKKERVYVYCINNKMCC-Z_{C14}
(m)hBD-18
   Z_{N15}-CWNRSGHCRKQCKDGEAVKDTCKNLRACC-Z_{C15}
(n) hBD-19
   Z_{N16}-CLMGLGRCRDHCNVDEKEIQKCKMKKCC-Z_{c16}
(o) hBD-20
   Z_{N17}-CWMDGHCRLLCKDGEDSIIRCRNRKRCC-Z_{C17}
(p) Z_{N}Z_{C}hBD-22
   Z_{N19}-CMGNSGICRASCKKNEQPYLYCRNCQSCC-Z_{C19}
(q) hBD-23
   Z_{N20}-CWKGQGACQTYCTRQETYMHLCPDASLCC-Z_{C20}
(r) hBD-24
   Z_{N21}-CELYQGMCRNACREYEIQYLTCPNDQKCC-Z_{C21}
(s) hBD-25
   Z_{N22}-CWIIKGHCRKNCKPGEQVKKPCKNGDYCC-Z_{C22}
(t) hBD-26
   Z_{N23}-CYYGTGRCRKSCKEIERKKEKCGEKHICC-Z_{C23}
(u) hBD-27
   Z_{N24}-CLGLPKCWNYRCEPLHLAYAFYCLLPTSCC-Z_{C24}
(v) hBD-28
   Z_{N25}-CVSNTPGYCRTCCHWGETALFMCNASRKCC-Z_{C25}
(w)hBD-29
   Z_{N26}-CWKNNVGHCRRRCLDTERYILLCRNKLSCC-Z_{C26}
(x) hBD-30
   Z_{N27}-CFNKVTGYCRKKCKVGERYEIGCLSGKLCC-Z_{C27}
(y) hBD-31
   Z_{N28}-CLNDVGICKKKCKPEEMHVKNGWAMCGKQRDCC-Z_{C28}
(z) hBD-32
   Z_{N29}-CWNFRGSCRDECLKNERVYVFCVSGKLCC-Z_{C29}
wobei

Z_{N2} einen Aminosäurerest oder einen Peptidrest von bis zu 30 Aminosäuren, insbesondere den Peptidrest IINTLQKYY und seine N-terminal verkürzten Fragmente, bedeutet,
Z_{C2} einen Aminosäurerest oder einen Peptidrest von bis zu 30 Aminosäuren, insbesondere den Peptidrest RRKK und seine C-terminal verkürzten Fragmente, bedeutet,
Z_{N3} einen Aminosäurerest oder einen Peptidrest von bis zu 30 Aminosäuren, insbesondere den Peptidrest EFELDRI und seine N-terminal verkürzten Fragmente, bedeutet,
Z_{C3} einen Aminosäurerest oder einen Peptidrest von bis zu 30 Aminosäuren, insbesondere den Peptidrest LRKWDESLLNRTKP und seine C-terminal verkürzten Fragmente, bedeutet,
Z_{N4} einen Aminosäurerest oder einen Peptidrest von bis zu 30 Aminosäuren, insbesondere den Peptidrest LKVVD und seine N-terminal verkürzten Fragmente, bedeutet,
Z_{C4} einen Aminosäurerest oder einen Peptidrest von bis zu 30 Aminosäuren, insbesondere den Peptidrest LH, bedeutet,
Z_{N5} einen Aminosäurerest oder einen Peptidrest von bis zu 30 Aminosäuren, insbesondere den Peptidrest EFAVCES und seine N-terminal verkürzten Fragmente, bedeutet,
Z_{C5} einen Aminosäurerest oder einen Peptidrest von bis zu 30 Aminosäuren, insbesondere den Peptidrest RQRI und seine C-terminal verkürzten Fragmente, bedeutet,
Z_{N7} einen Aminosäurerest oder einen Peptidrest von bis zu 30 Aminosäuren, insbesondere den Peptidrest NTI und seine N-terminal verkürzten Fragmente, bedeutet,
Z_{C7} einen Aminosäurerest oder einen Peptidrest von bis zu 30 Aminosäuren, insbesondere den Peptidrest VSNTDEEGKEKPEMD und seine C-terminal verkürzten Fragmente, bedeutet,
Z_{N8} einen Aminosäurerest oder einen Peptidrest von bis zu 30 Aminosäuren, insbesondere den Peptidrest GKFKEI und seine N-terminal verkürzten Fragmente, bedeutet,
Z_{C8} einen Aminosäurerest oder einen Peptidrest von bis zu 30 Aminosäuren, insbesondere den Peptidrest LPLGHQPRIEST und seine C-terminal verkürzten Fragmente, bedeutet,
Z_{N9} einen Aminosäurerest oder einen Peptidrest von bis zu 30 Aminosäuren, insbesondere den Peptidrest NAFFDEK und seine N-terminal verkürzten Fragmente, bedeutet,
Z_{C9} einen Aminosäurerest oder einen Peptidrest von bis zu 30 Aminosäuren, insbesondere den Peptidrest RTIQP und seine C-terminal verkürzten Fragmente, bedeutet,
Z_{N10} einen Aminosäurerest oder einen Peptidrest von bis zu 30 Aminosäuren, insbesondere den Peptidrest DLGPVEGH und seine N-terminal verkürzten Fragmente, bedeutet,
Z_{C10} einen Aminosäurerest oder einen Peptidrest von bis zu 30 Aminosäuren, insbesondere den Peptidrest RTWWIL und seine C-terminal verkürzten Fragmente, bedeutet,
Z_{N11} einen Aminosäurerest oder einen Peptidrest von bis zu 30 Aminosäuren, insbesondere den Peptidrest EVMK und seine N-terminal verkürzten Fragmente, bedeutet,
Z_{C11} einen Aminosäurerest oder einen Peptidrest von bis zu 30 Aminosäuren, insbesondere den Peptidrest VDPKYVPVKPKL und seine C-terminal verkürzten Fragmente, bedeutet,
Z_{N12} einen Aminosäurerest oder einen Peptidrest von bis zu 30 Aminosäuren, insbesondere den Peptidrest RIET und seine N-terminal verkürzten Fragmente, bedeutet,
Z_{C12} einen Aminosäurerest oder einen Peptidrest von bis zu 30 Aminosäuren, insbesondere den Peptidrest LKPKDQPHLPQHIKN und seine C-terminal verkürzten Fragmente, bedeutet,
Z_{N13} einen Aminosäurerest oder einen Peptidrest von bis zu 30 Aminosäuren, insbesondere den Peptidrest TEQLKK und seine N-terminal verkürzten Fragmente, bedeutet,
Z_{C13} einen Aminosäurerest oder einen Peptidrest von bis zu 30 Aminosäuren, insbesondere den Peptidrest LNIKELEA und seine C-terminal verkürzten Fragmente, bedeutet,
Z_{N14} einen Aminosäurerest oder einen Peptidrest von bis zu 30 Aminosäuren, insbesondere den Peptidrest TPGGTQR und seine N-terminal verkürzten Fragmente, bedeutet,
Z_{C14} einen Aminosäurerest oder einen Peptidrest von bis zu 30 Aminosäuren, insbesondere den Peptidrest VKPKYQPKERWWPF und seine C-terminal verkürzten Fragmente, bedeutet,
Z_{N15} einen Aminosäurerest oder einen Peptidrest von bis zu 30 Aminosäuren, insbesondere den Peptidrest PAYSGEKK und seine N-terminal verkürzten Fragmente, bedeutet,
Z_{C15} einen Aminosäurerest oder einen Peptidrest von bis zu 30 Aminosäuren, insbesondere den Peptidrest IPSNEDHRRV und seine C-terminal verkürzten Fragmente, bedeutet,
Z_{N16} einen Aminosäurerest oder einen Peptidrest von bis zu 30 Aminosäuren, insbesondere den Peptidrest FIGLRR und seine N-terminal verkürzten Fragmente, bedeutet,
Z_{C16} einen Aminosäurerest oder einen Peptidrest von bis zu 30 Aminosäuren, insbesondere den Peptidrest VGPKVVKLIK und seine C-terminal verkürzten Fragmente, bedeutet,
Z_{N17} einen Aminosäurerest oder einen Peptidrest von bis zu 30 Aminosäuren, insbesondere den Peptidrest VE, bedeutet,
Z_{C17} einen Aminosäurerest oder einen Peptidrest von bis zu 30 Aminosäuren, insbesondere den Peptidrest VPSR und seine C-terminal verkürzten Fragmente, bedeutet,
Z_{N19} einen Aminosäurerest oder einen Peptidrest von bis zu 30 Aminosäuren, insbesondere den Peptidrest HILR und seine N-terminal verkürzten Fragmente, bedeutet,
Z_{C19} einen Aminosäurerest oder einen Peptidrest von bis zu 30 Aminosäuren, insbesondere den Peptidrest LQSYMR und seine C-terminal verkürzten Fragmente, bedeutet,
Z_{N20} einen Aminosäurerest oder einen Peptidrest von bis zu 30 Aminosäuren, insbesondere den Peptidrest EFKR und seine N-terminal verkürzten Fragmente, bedeutet,
Z_{C20} einen Aminosäurerest oder einen Peptidrest von bis zu 30 Aminosäuren, insbesondere den Peptidrest LSYALK und seine C-terminal verkürzten Fragmente, bedeutet,
Z_{N21} einen Aminosäurerest oder einen Peptidrest von bis zu 30 Aminosäuren, insbesondere den Peptidrest PWNP und seine N-terminal verkürzten Fragmente, bedeutet,
Z_{C21} einen Aminosäurerest oder einen Peptidrest von bis zu 30 Aminosäuren, insbesondere den Peptidrest LKLSVK und seine C-terminal verkürzten Fragmente, bedeutet,
Z_{N22} einen Aminosäurerest oder einen Peptidrest von bis zu 30 Aminosäuren, insbesondere den Peptidrest QKS und seine N-terminal verkürzten Fragmente, bedeutet,
Z_{C22} einen Aminosäurerest oder einen Peptidrest von bis zu 30 Aminosäuren, insbesondere den Peptidrest IPSNTDS und seine C-terminal verkürzten Fragmente, bedeutet,
Z_{N23} einen Aminosäurerest oder einen Peptidrest von bis zu 30 Aminosäuren, insbesondere den Peptidrest GWIRR und seine N-terminal verkürzten Fragmente, bedeutet,
Z_{C23} einen Aminosäurerest oder einen Peptidrest von bis zu 30 Aminosäuren, insbesondere den Peptidrest VPKEKDK und seine C-terminal verkürzten Fragmente, bedeutet,
Z_{N24} einen Aminosäurerest oder einen Peptidrest von bis zu 30 Aminosäuren, insbesondere den Peptidrest QSS und seine N-terminal verkürzten Fragmente, bedeutet,
Z_{C24} einen Aminosäurerest oder einen Peptidrest von bis zu 30 Aminosäuren, insbesondere den Peptidrest LE, bedeutet,
Z_{N25} einen Aminosäurerest oder einen Peptidrest von bis zu 30 Aminosäuren, insbesondere den Peptidrest GSK und seine N-terminal verkürzten Fragmente, bedeutet,
Z_{C25} einen Aminosäurerest oder einen Peptidrest von bis zu 30 Aminosäuren, insbesondere den Peptidrest ISYSFLPK, bedeutet,
Z_{N26} einen Aminosäurerest oder einen Peptidrest von bis zu 30 Aminosäuren, insbesondere den Peptidrest FEPQK und seine N-terminal verkürzten Fragmente, bedeutet,
Z_{C26} einen Aminosäurerest oder einen Peptidrest von bis zu 30 Aminosäuren, insbesondere den Peptidrest ISIISHEY, bedeutet,
Z_{N27} einen Aminosäurerest oder einen Peptidrest von bis zu 30 Aminosäuren, insbesondere den Peptidrest LKK und seine N-terminal verkürzten Fragmente, bedeutet,
Z_{C27} einen Aminosäurerest oder einen Peptidrest von bis zu 30 Aminosäuren, insbesondere den Peptidrest ANDEEEK, bedeutet,
Z_{N28} einen Aminosäurerest oder einen Peptidrest von bis zu 30 Aminosäuren, insbesondere den Peptidrest WYVKK und seine N-terminal verkürzten Fragmente, bedeutet,
Z_{C28} einen Aminosäurerest oder einen Peptidrest von bis zu 30 Aminosäuren, insbesondere den Peptidrest VPADR, bedeutet,
Z_{N29} einen Aminosäurerest oder einen Peptidrest von bis zu 30 Aminosäuren, insbesondere den Peptidrest IET und seine N-terminal verkürzten Fragmente, bedeutet,
Z_{C29} einen Aminosäurerest oder einen Peptidrest von bis zu 30 Aminosäuren, insbesondere den Peptidrest LK, bedeutet,
und die zyklischen, amidierten, acetylierten, sulfatierten, phosphorylierten, glycosylierten, und oxydierten Derivate sowie Peptidfragmente, die aus den oben beschriebenen Aminosäuresequenzen abgeleitet werden.

Für die oben beschriebenen neuen Defensinpeptide wurden folgende kodierende Nukleinsäuresequenzen (cDNAs) gefunden, die auch Gegenstand der vorliegenden Erfindung sind:
(a) hBD-5 
ATGAGGATCCATTATCTTCTGTTTGCTTTGCTCTTCCTGTTTTTGGTGCCTG
TTCCAGGTCATGGAGGAATCATAAACACATTACAGAAATATTATTGCAGAG
TCAGAGGCGGCCGGTGTGCTGTGCTCAGCTGCCTTCCAAAGGAGGAACA
GATCGGCAAGTGCTCGACGCGTGGCCGAAAATGCTGCCGAAGAAAGAAA
(b) hBD-6 
CGAATTTGAATTGGACAGAATATGTGGTTATGGGACTGCCCGTTGCCGGA
AGAAATGTCGCAGCCAAGAATACAGAATTGGAAGATGTCCCAACACCTAT
GCATGCTGTTTGAGAAAATGGGATGAGAGCTTACTGAATCGTACAAAACC
C
(c) hBD-7 
ATTTAAAAGTTGTTGACTGCAGGAGAAGTGAAGGCTTCTGCCAAGAATAC
TGTAATTATATGGAAACACAAGTAGGCTACTGCTCTAAAAAGAAAGACGC
CTGCTGTTTACATTAAAACTGATGTTGC
(d) hBD-8 
TTTGCTGTCTGTGAGTCGTGCAAGCTTGGTCGGGGAAAATGCAGGAAGG
AGTGCTTGGAGAATGAGAAGCCCGATGGAAATTGCAGGCTGAACTTTCTC
TGCTGCAGACAGAGGATC
(e) hBD-10 
AAATACCATCTGCCGTATGCAGCAAGGGATCTGCAGACTTTTTTCTGCCA
TTCTGGTGAGAAAAAGCGTGACATTTGCTCTGATCCCTGGAATAGGTGTT
GCGTATCAAATACAGATGAAGAAGGAAAAGAGAAACCAGAGATGGATGG
CAGATCTGGGATCTAAAATATAAGCTCCC
(f) hBD-11 
AGGGGAGCGGGCTACTCACCTCCAGCCTTTTGTCATCCAGGGGCAAATTC
AAGGAGATCTGTGAACGTCCAAATGGCTCCTGTCGGGACTTTTGCCTCGA
AACAGAAATCCATGTTGGGAGATGTTTAAATAGCCGACCCTGCTGCCTGC
CTCTGGGGCATCAACCAAGAATTGAGAGCACTACACCCAAAAAGGAC
(g) hBD-12 
CTCAAGACCCACCCCAGTCATGAGGACTTTCCCTTTTCTCTTTGCCGTGCT
CTTCTTTCTGACCCCAGCCAAGAATGCATTTTTTGATGAGAAATGCAACAA
ACTTAAAGGGACATGCAAGAACAATTGCGGGAAAAATGAAGAACTTATTG
CTCTCTGCCAGAAGTCTCTGAAATGCTGTCGGACCATCCAGCCATGTGGG
AGCATTATAGAT
(h) hBD-13 
GTGATTTGGGTCCTGTGGAAGGTCATTGTCTCAATTTGTCTGGTGTTTGCA
GAAGAGATGTCTGCAAAGTAGTAGAAGATCAAATTGGTGCCTGCCGAAGA
AGGATGAAGTGTTGTAGAACATGGTGGATTTTAATGCCAATTCCAACACC
ACTTATCATGTCAGATTATCAAGAACCCCTTAAACATAAGTTGAAA
(i) hBD-14 
GAAGTCATGAAATGTTGGGGCAAGTCAGGCAGGTGCAGAACAACATGTA
AAGAAAGTGAAGTATACTATATATTATGCAAAACTGAGGCTAAGTGCTGTG
TGGATCCCAAGTATGTACCTGTAAAACCAAAATTAACAGACACAAATACAA
GCCTGGAATCAACTTCTGCAGTCTGACACCTCTCTTCCAACCTTGAGTCTC
AACATCATGGGATCCTGCAGTTCTAT
(j) hBD-15 
GCAGGATTGAAACATGTTGGAATTTTCGTGGCTCCTGCCGTGACGAATGC
CTGAAGAATGAAAGGGTCTATGTTTTCTGCGTGAGTGGTAAACTGTGCTG
TTTGAAGCCCAAGGACCAGCCACATTTACCACAGCATATAAAGAAT
(k) hBD-16 
TGAGGAAGGTAGCATAGTGTGCAGTTCACTGGACCAAAAGCTTTGGCTGC
ACCTCTTCTGGAAAGCTGGCCATGGGGTCTTCATGATCATTGCAATTCTGC
TGTTCCAGAAACCCACAGTAACCGAACAACTTAAGAAGTGCTGGAATAAC
TATGTACAAGGACATTGCAGGAAAATCTGCAGAGTAAATGAAGTGCCTGA
GGCACTATGTGAAAATGGGAGATACTGTTGCCTCAATATCAAGGAACTGG
AAGCATGTAAAAAAATTACAAAGCCACCTCGTCCAAAGCCAGCAACACTT
GCACTGACTCTTCAAGACTATGTTACAATAATAGAAAATTTCCCAAGCCTG
AAGACACAGTCTACA
(l) hBD-17 
GGACTTGCAGCTTCATTTTGGGCTGCCTTAGCCATGAAGCTCCTTTTGCTG
ACTTTGACTGTGCTGCTGCTCTTATCCCAGCTGACTCCAGGTGGCACCCA
AAGATGCTGGAATCTTTATGGCAAATGCCGTTACAGATGCTCCAAGAAGG
AAAGAGTCTATGTTTACTGCATAAATAATAAAATGTGCTGCGTGAAGCCCA
AGTACCAGCCAAAAGAAAGGTGGTGGCCATTT
(m)hBD-18 
TTCCCAAGGACCATGAAACTCCTGCTGCTGGCTCTTCCTATGCTTGTGCTC
CTACCCCAAGTGATCCCAGCCTATAGTGGTGAAAAAAAATGCTGGAACAG
ATCAGGGCACTGCAGGAAACAATGCAAAGATGGAGAAGCAGTGAAAGAT
ACATGCAAAAATCTTCGAGCTTGCTGCATTCCATCCAATGAAGACCACAG
GCGAGTTCCTGCGACATCTCCCACACCCTTGAGTGACTCAACACCAGGAA
TTATTGATGATATTTTAACAGTAAGGTTCACGACAGACTACTTTGAAGTAA
 
GCAGCAAGAAAGATATGGTTGAAGAGTCTGAGGCGGGAAGGGGAACTGA
GACCTCTCTTCCAAATGTTCACCATAGCTCA
(n) hBD-19 
ACCATGAAGCTCCTTTTCCTATCTTTGCCAGCCTCATGCTACAGTACCAG
GTGAACACAGAATTTATTGGCTTGAGACGCTGTTTAATGGGTTTGGGGAG
ATGCAGGGATCACTGCAATGTGGATGAAAAAGAGATACAGAAATGCAAGA
TGAAAAAATGTTGTGTTGGACCAAAAGTGGTTAAATTGATTAAAAACTACC
TACAATATGGAACACCAAATGTACTTAATGAAGACGTCCAAGAAATGCTAA
AACCTGCCAAGAATTCTAGTGCTGTGATACAAAGAAAACATATTTTATCTG
TTCTCCCCCAAATCAAAAGCACTAGCTTTTTTGCTAATACCAACTTTGTCAT
CATTCCAAATGCCACCCCTATGAACTCTGCCACCATCAGCACTATGACCCC
AGGACAGATCACATACACTGCTACTTCTACCAAGAGTAACACCAAAGAAA
GCAGAGATTCTGCCACTGCCTCGCCACCACCAGCACCACCTCCACCAAAC
ATACTGCCAACACCATCACTGGAGCTAGAGGAAGCAGAAGAGCAG
(o) hBD-20 
TAGAGTGTTGGATGGATGGACACTGCCGGTTGTTGTGCAAAGATGGTGAA
GACAGCATCATACGCTGCCGAAATCGTAAACGGTGCTGTGTTCCTAGTCG
TTATTTAACAATCCAACCAGTAACAATTCATGGAATCCTTGGCTGGACCAC
TCCTCAGATGTCCACAACAGCTCCAAAAATGAAGACAAATATAACTAATAG
ATAGAAA
(p) hBD-22 
AGCAAAGCTCATCTCTGCCGTGCTGCAGGGAACCCTATTTCCTTCCCCTG
CAGCTCAGCCACCTCCTCCTCTCAGGTCTGCCAGCCATGAAACTTCTTTAC
CTGTTTCTTGCCATCCTTCTGGCCATAGAAGAACCAGTGATATCAGGCAAA
CGCCACATCCTTCGATGCATGGGTAACAGTGGAATTTGTAGGGCCTCTTG
CAAAAAGAACGAACAGCCCTACCTCTATTGCAGAAATTGTCAGTCCTGCT
 
GCCTCCAGTCCTACATGAGGATAAGCATTTCTGGCAAAGAGGAAAATACC
GACTGGTCTTATGAGAAGCAGTGGCCAAGACTACCT
(q) hBD-23 
TGAATTCAAACGGTGCTGGAAGGGTCAAGGGGCCTGCCAAACTTACTGCA
CAAGGCAAGAAACTTACATGCACCTGTGCCCGGATGCGTCCCTGTGCTGT
CTCTCCTATGCATTGAAACCTCCACCGGTCCCCAAGCATGAATATGAG
(r) hBD-24 
CCTTGGAATCCATGTGAGCTTTACCAAGGCATGTGCAGAAACGCCTGCAG
AGAATATGAAATCCAATACTTAACCTGCCCAAATGATCAAAAGTGCTGCCT
GAAACTTTCTGTGAAAATAACCAGTTCTAAAAATGTGAAGGAGGATTACGA
CTCTAACTCCAACTTGTCAGTTACAAACAGTTCAAGCTACTCTCACATT
(s) hBD-25 
CCAAAAATCTTGCTGGATCATAAAAGGACACTGCAGGAAAAACTGCAAAC
CTGGTGAACAGGTTAAAAAGCCATGTAAAAATGGTGACTATTGCTGCATT
CCAAGCAACACAGATTCT
(t) hBD-26 
ATGGATGGATCAGAAGGTGCTATTATGGAACTGGCAGATGCAGGAAATCA
TGCAAAGAAATTGAGAGGAAGAAAGAAAAATGTGGGAAAAACATATTTG
CTGTGTCCCTAAAGAAAAGGATAAACTATCACACATTCACGACCAAAAAGA
GACAAGTGAGCTATATATC
(u) hBD-27 
CAATCCTCCTGCCTTGGCCTCCCAAAGTGCTGGAATTATAGGTGTGAGCC
ACTGCACCTGGCCTATGCCTTTTATTGCCTCCTGCCTACCTCCTGCTGTTT
GGAATGTGAAAGCAAGACTGGAGCTCTACCTTGGACTATGAAAAACAAGG
ACCTCACC
(v) hBD-28 
GGGTCAAAATGTGTGAGTAACACCCCAGGATACTGCAGGACATGTTGCCACTGG
GGGGAGACAGCATTGTTCATGTGCAACGCTTCCAGAAAATGCTGCATCAGCTACT
CCTTCCTGCCGAAGCCTGACCTACCACAGCTCATCGGTAACCACTGGCAATCAAG
GAGAAGAAACACACAAAGGAAAGACAAGAAGCAACAAACGACCGTAACATCA
(w)hBD-29 
TTTGAACCCCAAAAATGTTGGAAGAATAATGTAGGACATTGCAGAAGACGATGTT
TAGATACTGAAAGGTACATACTTCTTTGTAGGAACAAGCTATCATGCTGCATTTCT
ATAATATCACATGAATATACTCGACGACCAGCATTTCCTGTGATTCACCTAGAGGA
TATAACATTGGATTATAGTGATGTGGACTCTTTTACTGGTTCCCCAGTATCTATGT
TGAATGATCTGATAACATTTGACACAACTAAATTTGGAGAAACCATGACACCTGA
GACCAATACTCCTGAGACTACTATGCCACCATCTGAGGCCACTACTCCCGAGACT
ACTATGCCACCATCTGAGACTGCTACTTCCGAGACTATGCCACCACCTTCTCAGA
CAGCTCTTACTCATAAT
(x) hBD-30 
CTCAAAAAATGCTTCAATAAAGTAACAGGCTATTGCAGGAAGAAATGCAAGG
TAGGAGAAAGATATGAAATAGGATGTCTAAGTGGGAAATTATGTTGTGCTAA
TGATGAAGAAGAGAAAAAACATGTGTCATTTAAGAAGCCACATCAACATTCT
GGTGAGAAGCTGAGTGTGCTGCAGGATTACATCATCTTACCCACCATCACCA
TTTTCACAGTC
(y) hBD-31 
ATGAAGTCCCTACTGTTCACCCTTGCAGTTTTTATGCTCCTGGCCCAATTGG
TCTCAGGTAATTGGTATGTGAAAAAGTGTCTAAACGACGTTGGAATTTGCAA
GAAGAAGTGCAAACCTGAAGAGATGCATGTAAAGAATGGTTGGGCAATGTG
CGGCAAACAAAGGGACTGCTGTGTTCCAGCTGACAGACGTGCTAATTATCC
TGTTTTCTGTGTCCAGACAAAGACTACAAGAATTTCAACAGTAACAGCAACA
ACAGCAACAACAACTTTGATGATGACTACTGCTTCGATGTCTTCGATGGCTC
CTACCCCCGTTTCTCCCACTGGT
(z) hBD-32

ATTGAAACATGTTGGAATTTTCGTGGCTCCTGCCGTGACGAATGCCTGAAGA
ATGAAAGGGTCTATGTTTTCTGCGTGAGTGGTAAACTGTGCTGTTTGAAGCC
CAAGGACCAGCCACATTTACCACAGCATATAAAGAAT

Während durch die Analyse der entsprechenden kodierenden Nukleotidsequenzen die Gene der neuen Defensinpeptide hBD-5, hBD-6, hBD-7, hBD-8, hBD-10, hBD-11, hBD-12und hBD-13, auf Chromosom 8 gefunden wurden, konnten die Gene der erfindungsgemäßen neuen Defensinpeptide hBD-14, hBD-15, hBD-16, hBD-17, hBD-18, hBD-19, hBD-20, hBD-22, hBD-23, hBD-24, hBD-25, hBD-26, hBD-27, hBD-28, hBD-29, hBD-30, hBD-31 und hBD-32 überraschenderweise Chromosom 20 zugeordnet werden.

Damit ist weiter Aufgabe der vorliegenden Erfindung, die neuen Peptide hBD-5 bis hBD-32 bereitzustellen, die dadurch gekennzeichnet sind, daß diese jeweils als ein gut zugängliches Arzneimittel mit biologisch und therapeutischer Aktivität eines natürlichen Stoffes verwanden werden können.

Die vorliegende Erfindung stellt des weiteren ein Herstellungsverfahren für die erfindungsgemäßen Peptide sowie die Verwendung der erfindungsgemäßen Peptide als Arzneimittel für verschiedene therapeutische und diagnostische Indikationen bereit. Dazu können die Defensinpeptide als hochreine Stoffe oder - wenn für die bestimmte Verwendung ausreichend - innerhalb eines teilweise aufgereinigten Peptidgemisches oder als Gemisch mehrerer der erfindungsgemäßen hochreinen Defensinpeptide verwandt werden.

Die erfindungsgemäßen Peptide können eingesetzt werden zur Behandlung von Erkrankungen, die bei bakteriellen Organbesiedlungen entstehen.

Die erfindungsgemäßen Peptide sind weiterhin einsetzbar zur Behandlung von Erkrankungen des menschlichen Organismus, insbesondere mit Beteiligung des Magen-Darm-Traktes, der Atemwege und des Urogenitalapparates.

Die erfindungsgemäßen Peptide können in einer weiteren Ausgestaltung der Erfindung eingesetzt werden zur Behandlung von Erkrankungen des menschlichen Organismus, insbesondere mit Beteiligung des Intugementes und seiner Anhangsdrüsen.
Die erfindungsgemäßen Peptide können auch eingesetzt werden zur Behandlung von Systemerkrankungen bei Überproduktion oder Mangel der Defensinpeptide, insbesondere durch gegen die Defensinpeptide gebildete Antikörper oder zur Verwendung in der Substitutionstherapie.

In einer weiteren Ausführungsform der Erfindung können die erfindungsgemäßen Peptide zur Behandlung von chronischen Erkrankungen, teils vergesellschaftet mit den bereits erwähnten Erkrankungen eingesetzt werden, indem diese in geeigneter Form für die Behandlung benutzt werden.

Die erfindungsgemäßen Peptide können weiterhin eingesetzt werden zur Behandlung von Erkrankungen im akuten Stadium.

Die erfindungsgemäßen Peptide können eingesetzt werden zur Behandlung der Störung der Fertilität, insbesondere bei Krankheiten der mit Oocyten verbundenen Spermienpenetrationsstörungen und Inidationsstörungen sowie Maturationsstörungen im männlichen Reproduktionsapparates, sowie als Kontrazeptivum.

Die erfindungsgemäßen Peptide können eingesetzt werden zur Diagnose der bereits erwähnten Erkrankungen, indem beispielsweise Antikörper gegen eines oder mehrere der erfindungsgemäßen Peptide oder seiner Derivate oder ihrer Fragmente hergestellt werden und die Blutkonzentration eines oder mehrerer der erfindungsgemäßen Peptide über immunologische Verfahren gemessen wird.

Die vorliegende Erfindung betrifft weiter verschiedene Verfahren zur Herstellung der erfindungsgemäßen neuen Defensinpeptide oder ihrer Derivate dadurch gekennzeichnet, dass dieses über eine prokaryontische oder eine eukaryontische Expression hergestellt und chromatographisch gereinigt werden, sowie ein weiteres Verfahren zur Herstellung der Defensinpeptide ihrer Derivate, indem man sie aus menschlichem Blut über Chromatographie-Verfahren in bekannter Weise isoliert, und schließlich ein Verfahren zur Herstellung der Defensinpeptide oder ihrer Derivate, indem man diese Defensinpeptide durch die üblichen Verfahren der Festphasen- und Flüssigphasen-Synthese aus den geschützten Aminosäuren, die in der angegebenen Sequenz enthalten sind, herstellt, deblockiert und es mit den gängigen Chromatographie-Verfahren reinigt.

Die Defensinpeptide werden chemisch synthetisiert und als Arzneimittel zubereitet. Auch die gentechnologische Herstellung durch Verwendung übliche Vektoren ist erarbeitet. Auf diesem Wege wird die neuen Defensinpeptide sowohl (1) in prokaryontischen als auch (2) in eukaryontischen Organismen hergestellt. Hierfür stehen verschiedene Expressionsvektoren routinemässig zur sekretorischen oder direkten cytoplasmatischen Expression zur Verfügung.

Die Arzneimittelzubereitungen enthalten eines oder mehrere der erfindungsgemäßen neuen Defensinpeptide oder ein physiologisch verträgliches Salz dieser Peptide. Die Form und Zusammensetzung der Arzneimittel, welche eines oder mehrere der neuen Defensinpeptide enthalten, richtet sich nach der Art der Verabreichung. Die Arzneimittel eines oder mehrere der neuen Defensinpeptide enthaltend können parenteral, intranasal, oral und mittels Inhalation verabreicht werden. Vorzugsweise werden diese Arzneimittel enthaltend eines oder mehrere der neuene Defensinpeptide mit einem Injektionspräparat, entweder als Lösung oder als Lyophilisat zur Auflösung unmittelbar vor Gebrauch konfektioniert. Die Arzneimittelzubereitungen können außerdem Hilfsstoffe enthalten, die abfülltechnisch bedingt sind, einen Beitrag zur Löslichkeit, Stabilität oder Sterilität des Arzneimittels leisten oder den Wirkungsgrad der Aufnahme in den Körper erhöhen.

Die zu verabreichende Tagesdosis für die erfindungsgemäßen Defensinpeptide hängt von der Indikation und der Anwendung bestimmter Derivate ab. Bei i.v./i.m. Injektion liegt sie im Bereich von 100 bis 1200 Einheiten (µg)/Tag, bei täglicher subcutaner Injektion vorzugsweise bei 300 - 2400 Einheiten (µg)/Tag.

Die Bestimmung der biologischen Aktivität für die erfindungsgemäßen neuen Defensinpeptide basiert auf Messungen gegen international gebräuchliche Referenzpräparationen für antibiotische Substanzen. Die erfindungsgemäßen neuen Defensinpeptide hBD-5, hBD-6, hBD-7, hBD-8, hBD-10, hBD-11, hBD-12, hBD-13, hBD-14, hBD-15, hBD-16, hBD-17, hBD-18, hBD-19, hBD-20, hBD-22, hBD-23, hBD-24, hBD-25, hBD-26, hBD-27, hBD-28, hBD-29, hBD-30, hBD-31und hBD-32, sind dadurch gekennzeichnet, dass sie sich besonders auch für die LangzeitTherapie bei Infektionserkrankungen eignen, da sie über eine ausgezeichnete biologische Wirksamkeit verfügen und andererseits auch bei Dauerbehandlung keine Immunreaktion auslösen.

Aufgrund der biologischen Wirkung der erfindungsgemäßen Defensinpeptide ist gezeigt, dass die erfindungsgemäßen Präparate weiter als Mittel zur Therapie von infektiösen Erkrankungen vieler Epithelorgane anwendbar sind.

Zur Bestimmung der Aktivität wurden beispielhaft die Peptide hBD10, hBD17 und hBD19 auf ihre antimikrobielle Wirkung hin getestet. Im Radial-Diffusions-Assay konnten die in Tabelle 1 angegebenen Aktivitäten der Peptide gegen verschiedene Bakterienstämme gemessen werden. Dabei bedeutet (+) die Bildung eines Hemmhofes und (-) keine Bildung eines Hemmhofs.Tabelle 1

| | hBD10 | hBD17 | hBD19 |
|---|---|---|---|
| Escherichia coli | (+) | (+) | (+) |
| Staphylococcus carnosus | (+) | (+) | (+) |
| Saccharomyces cerevisiae | (+) | (+) | (-) |

Für eine genauere Bestimmung der antibiotischen Aktivität wurde die minimale inhibitorische Konzentration (MIC) der o.g. Defensine nach Standardmethoden bestimmt. Die Befunde sind in Tabelle 2 angegeben, wobei die MIC-Werte Konzentrationen in [µg/ml] entsprechen (nd = nicht gemessen).Tabelle 2

| | hBD10 | hBD17 | hBD19 |
|---|---|---|---|
| Escherichia coli | nd | nd | nd |
| Staphylococcus carnosus | <50 | <25 | <25 |
| Saccharomyces cerevisiae | nd | nd | nd |

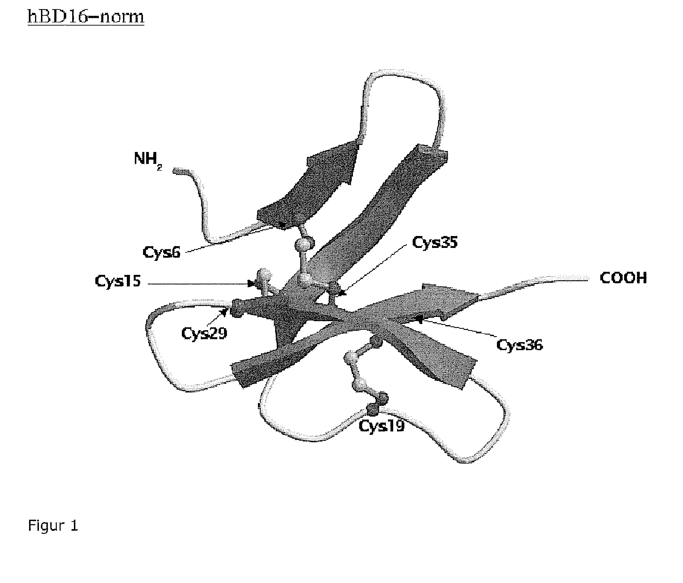

Weiterhin wurden Strukturanalysen mit hBD16 durchgeführt. Abbildung 1 zeigt die in Lösung gefundene NMR-Struktur von hBD16.

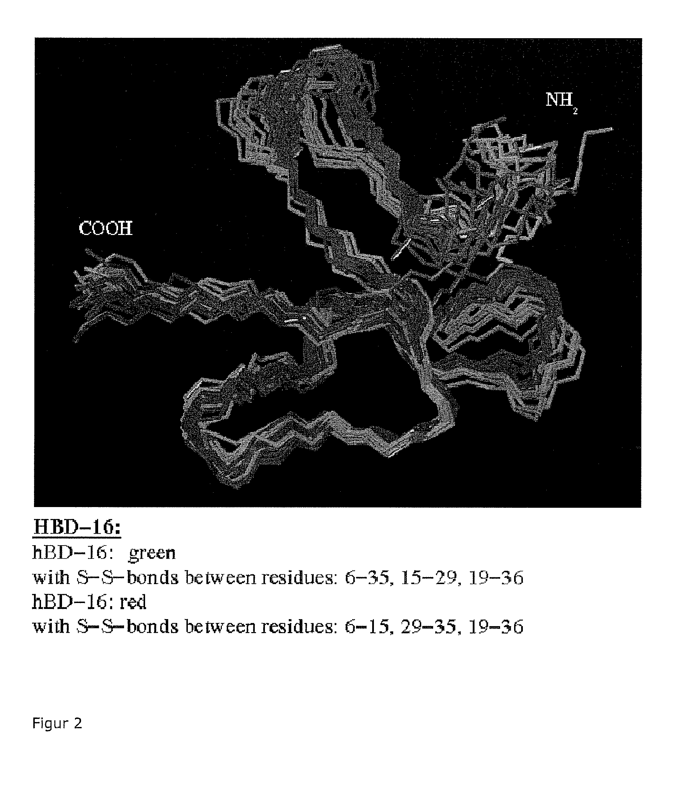

Die räumliche Lage der Cysteine Cys 6, 15, 29 und 35 zeigt, dass die Verbrückung dieser Positionen nicht zwingend eine Strukturveränderung bedeuten muss, die zu einer Verminderung der Aktivität führt. Dieses konnte anhand des Vergleichs zweier Verbrückungsmuster gezeigt werden (Figur 2).

## Patentansprüche

1. Peptide mit der Aminosäuresequenz
Z_{N}-C-Xₘ-X₁-X-C-X₂-Xₙ-C-X-X-X-X₃-Xₒ-C-Xₚ-C-C-Z_{c}
wobei Z_{N} ein Aminosäurerest oder ein Peptidrest von bis zu 30 Aminosäuren , Z_{C} ein Aminosäurerest oder ein Peptidrest von bis zu 30 Aminosäuren,
X = eine beliebige Aminosäure
Xₘ = 3-6 beliebige Aminosäuren
Xₙ = 2-3 Aminosäuren
Xₒ = 5-9 Aminosäuren
Xₚ = 4-6 Aminosäuren
X₁= G, A oder P
X₂= R, K, W, Q oder A ist
X₃= E oder H ist.

2. Peptid nach Anspruch 1 mit der Aminosäuresequenz
(cc) hBD-7
Z_{N4}-CRRSEGFCQEYCNYMETQVGYCSKKKDACC-Z_{C4}
Z_{N4} einen Aminosäurerest oder einen Peptidrest von bis zu 30 Aminosäuren, insbesondere den Peptidrest LKVVD und seine N-terminal verkürzten Fragmente und Z_{C4} einen Aminosäurerest oder einen Peptidrest von bis zu 30 Aminosäuren, insbesondere den Peptidrest LH, bedeutet.

3. Peptid nach Anspruch 1 mit der Aminosäuresequenz
(dd)hBD-8
Z_{N5}-CKLGRGKCRKECLENEKPDGNCRLNFLCC-Z_{C5}
Z_{N5} einen Aminosäurerest oder einen Peptidrest von bis zu 30 Aminosäuren, insbesondere den Peptidrest EFAVCES und seine N-terminal verkürzten Fragmente und Z_{C5} einen Aminosäurerest oder einen Peptidrest von bis zu 30 Aminosäuren, insbesondere den Peptidrest RQRI und seine C-terminal verkürzten Fragmente, bedeutet.

4. Peptid nach Anspruch 1 mit der Aminosäuresequenz
(ee) hBD-10
Z_{N7}-CHMQQGICRLFFCHSGEKKRGICSDPWNRCC-Z_{C7}
Z_{N7} einen Aminosäurerest oder einen Peptidrest von bis zu 30 Aminosäuren, insbesondere den Peptidrest NTI und seine N-terminal verkürzten Fragmente und Z_{C7} einen Aminosäurerest oder einen Peptidrest von bis zu 30 Aminosäuren, insbesondere den Peptidrest VSNTDEEGKEKPEMD und seine C-terminal verkürzten Fragmente, bedeutet.

5. Peptid nach Anspruch 1 mit der Aminosäuresequenz
(ff) hBD-11
Z_{N8}-CERPNGSCRDFCLETEIHVGRCLNSRPCC-Z_{C8}
Z_{N8} einen Aminosäurerest oder einen Peptidrest von bis zu 30 Aminosäuren, insbesondere den Peptidrest GKFKEI und seine N-terminal verkürzten Fragmente und Z_{C8} einen Aminosäurerest oder einen Peptidrest von bis zu 30 Aminosäuren, insbesondere den Peptidrest LPLGHQPRIEST und seine C-terminal verkürzten Fragmente, bedeutet.

6. Peptid nach Anspruch 1 mit der Aminosäuresequenz
(gg) hBD-12
Z_{N9}-CNKLKGTCKNNCGKNEELIALCQKSLKCC-Z_{C9}
Z_{N9} einen Aminosäurerest oder einen Peptidrest von bis zu 30 Aminosäuren, insbesondere den Peptidrest NAFFDEK und seine N-terminal verkürzten Fragmente und Z_{C9} einen Aminosäurerest oder einen Peptidrest von bis zu 30 Aminosäuren, insbesondere den Peptidrest RTIQP und seine C-terminal verkürzten Fragmente, bedeutet.

7. Peptid nach Anspruch 1 mit der Aminosäuresequenz
(hh)hBD-13
Z_{N10}-CLNLSGVCRRDVCKVVEDQIGACRRRMKCC-Z_{C10}
Z_{N10} einen Aminosäurerest oder einen Peptidrest von bis zu 30 Aminosäuren, insbesondere den Peptidrest DLGPVEGH und seine N-terminal verkürzten Fragmente und Z_{C10} einen Aminosäurerest oder einen Peptidrest von bis zu 30 Aminosäuren, insbesondere den Peptidrest RTWWIL und seine C-terminal verkürzten Fragmente, bedeutet.

8. Peptid nach Anspruch 1 mit der Aminosäuresequenz
(ii) hBD-14
Z_{N11}-CWGKSGRCRTTCKESEVYYlLCKTEAKCC-Z_{C11}
Z_{N11} einen Aminosäurerest oder einen Peptidrest von bis zu 30 Aminosäuren, insbesondere den Peptidrest EVMK und seine N-terminal verkürzten Fragmente und Z_{C11} einen Aminosäurerest oder einen Peptidrest von bis zu 30 Aminosäuren, insbesondere den Peptidrest VDPKYVPVKPKL und seine C-terminal verkürzten Fragmente bedeutet.

9. Peptid nach Anspruch 1 mit der Aminosäuresequenz
(jj) hBD-15
Z_{N12}-CWNFRGSCRDECLKNERVYVFCVSGKLCC-Z_{C12}
Z_{N12} einen Aminosäurerest oder einen Peptidrest von bis zu 30 Aminosäuren, insbesondere den Peptidrest RIET und seine N-terminal verkürzten Fragmente und Z_{C12} einen Aminosäurerest oder einen Peptidrest von bis zu 30 Aminosäuren, insbesondere den Peptidrest LKPKDQPHLPQHIKN und seine C-terminal verkürzten Fragmente bedeutet.

10. Peptid nach Anspruch 1 mit der Aminosäuresequenz
(kk)hBD-16
Z_{N13}-CWNNYVQGHCRKICRVNEVPEALCENGRYCC-Z_{C13}
Z_{N13} einen Aminosäurerest oder einen Peptidrest von bis zu 30 Aminosäuren, insbesondere den Peptidrest TEQLKK und seine N-terminal verkürzten Fragmente und Z_{C13} einen Aminosäurerest oder einen Peptidrest von bis zu 30 Aminosäuren, insbesondere den Peptidrest LNIKELEA und seine C-terminal verkürzten Fragmente bedeutet.

11. Peptid nach Anspruch 1 mit der Aminosäuresequenz
(11) hBD-17
Z_{N14}-CWNLYGKCRYRCSKKERVYVYCINNKMCC-Z_{C14}
Z_{N14} einen Aminosäurerest oder einen Peptidrest von bis zu 30 Aminosäuren, insbesondere den Peptidrest TPGGTQR und seine N-terminal verkürzten Fragmente und Z_{C14} einen Aminosäurerest oder einen Peptidrest von bis zu 30 Aminosäuren, insbesondere den Peptidrest VKPKYQPKERWWPF und seine C-terminal verkürzten Fragmente, bedeutet.

12. Peptid nach Anspruch 1 mit der Aminosäuresequenz
(mm)hBD-18
Z_{N15}-CWNRSGHCRKQCKDGEAVKDTCKNLRACC-Z_{C15}
Z_{N15} einen Aminosäurerest oder einen Peptidrest von bis zu 30 Aminosäuren, insbesondere den Peptidrest PAYSGEKK und seine N-terminal verkürzten Fragmente und Z_{C15} einen Aminosäurerest oder einen Peptidrest von bis zu 30 Aminosäuren, insbesondere den Peptidrest IPSNEDHRRV und seine C-terminal verkürzten Fragmente, bedeutet.

13. Peptid nach Anspruch 1 mit der Aminosäuresequenz
(nn)hBD-19
Z_{N16}-CLMGLGRCRDHCNVDEKEIQKCKMKKCC-Z_{C16}
Z_{N16} einen Aminosäurerest oder einen Peptidrest von bis zu 30 Aminosäuren, insbesondere den Peptidrest FIGLRR und seine N-terminal verkürzten Fragmente und Z_{C16} einen Aminosäurerest oder einen Peptidrest von bis zu 30 Aminosäuren, insbesondere den Peptidrest VGPKVVKLIK und seine C-terminal verkürzten Fragmente, bedeutet.

14. Peptid nach Anspruch 1 mit der Aminosäuresequenz
(oo)hBD-20
Z_{N17}-CWMDGHCRLLCKDGEDSIIRCRNRKRCC-Z_{C17}
Z_{N17} einen Aminosäurerest oder einen Peptidrest von bis zu 30 Aminosäuren, insbesondere den Peptidrest VE und Z_{C17} einen Aminosäurerest oder einen Peptidrest von bis zu 30 Aminosäuren, insbesondere den Peptidrest VPSR und seine C-terminal verkürzten Fragmente, bedeutet.

15. Peptid nach Anspruch 1 mit der Aminosäuresequenz
(pp)hBD-22
Z_{N19}-CMGNSGICRASCKKNEQPYLYCRNCQSCC-Z_{C19}
Z_{N19} einen Aminosäurerest oder einen Peptidrest von bis zu 30 Aminosäuren, insbesondere den Peptidrest HILR und seine N-terminal verkürzten Fragmente, bedeutet und Z_{C19} einen Aminosäurerest oder einen Peptidrest von bis zu 30 Aminosäuren, insbesondere den Peptidrest LQSYMR und seine C-terminal verkürzten Fragmente, bedeutet.

16. Peptid nach Anspruch 1 mit der Aminosäuresequenz
(qq)hBD-23
Z_{N20}-CWKGQGACQTYCTRQETYMHLCPDASLCC-Z_{C20}
Z_{N20} einen Aminosäurerest oder einen Peptidrest von bis zu 30 Aminosäuren, insbesondere den Peptidrest EFKR und seine N-terminal verkürzten Fragmente und Z_{C20} einen Aminosäurerest oder einen Peptidrest von bis zu 30 Aminosäuren, insbesondere den Peptidrest LSYALK und seine C-terminal verkürzten Fragmente, bedeutet.

17. Peptid nach Anspruch 1 mit der Aminosäuresequenz
(rr) hBD-24
Z_{N21}-CELYQGMCRNACREYEIQYLTCPNDQKCC-Z_{C21}
Z_{N21} einen Aminosäurerest oder einen Peptidrest von bis zu 30 Aminosäuren, insbesondere den Peptidrest PWNP und seine N-terminal verkürzten Fragmente und Z_{C21} einen Aminosäurerest oder einen Peptidrest von bis zu 30 Aminosäuren, insbesondere den Peptidrest LKLSVK und seine C-terminal verkürzten Fragmente, bedeutet.

18. Peptid nach Anspruch 1 mit der Aminosäuresequenz
(ss)hBD-25
Z_{N22}-CWIIKGHCRKNCKPGEQVKKPCKNGDYCC-Z_{C22}
Z_{N22} einen Aminosäurerest oder einen Peptidrest von bis zu 30 Aminosäuren, insbesondere den Peptidrest QKS und seine N-terminal verkürzten Fragmente und Z_{C22} einen Aminosäurerest oder einen Peptidrest von bis zu 30 Aminosäuren, insbesondere den Peptidrest IPSNTDS und seine C-terminal verkürzten Fragmente, bedeutet.

19. Peptid nach Anspruch 1 mit der Aminosäuresequenz
(tt) hBD-26
Z_{N23}-CYYGTGRCRKSCKEIERKKEKCGEKHICC-Z_{C23}
Z_{N23} einen Aminosäurerest oder einen Peptidrest von bis zu 30 Aminosäuren, insbesondere den Peptidrest GWIRR und seine N-terminal verkürzten Fragmente und Z_{C23} einen Aminosäurerest oder einen Peptidrest von bis zu 30 Aminosäuren, insbesondere den Peptidrest VPKEKDK und seine C-terminal verkürzten Fragmente, bedeutet.

20. Peptid nach Anspruch 1 mit der Aminosäuresequenz
(uu)hBD-27
Z_{N24}-CLGLPKCWNYRCEPLHLAYAFYCLLPTSCC-Z_{C24}
Z_{N24} einen Aminosäurerest oder einen Peptidrest von bis zu 30 Aminosäuren, insbesondere den Peptidrest QSS und seine N-terminal verkürzten Fragmente und Z_{C24} einen Aminosäurerest oder einen Peptidrest von bis zu 30 Aminosäuren, insbesondere den Peptidrest LE, bedeutet.

21. Peptid nach Anspruch 1 mit der Aminosäuresequenz
(vv)hBD-28
Z_{N25}-CVSNTPGYCRTCCHWGETALFMCNASRKCC-Z_{C25}
Z_{N25} einen Aminosäurerest oder einen Peptidrest von bis zu 30 Aminosäuren, insbesondere den Peptidrest GSK und seine N-terminal verkürzten Fragmente und Z_{C25} einen Aminosäurerest oder einen Peptidrest von bis zu 30 Aminosäuren, insbesondere den Peptidrest ISYSFLPK, bedeutet.

22. Peptid nach Anspruch 1 mit der Aminosäuresequenz
(ww)hBD-29
Z_{N26}-CWKNNVGHCRRRCLDTERYILLCRNKLSCC-Z_{C26}
Z_{N26} einen Aminosäurerest oder einen Peptidrest von bis zu 30 Aminosäuren, insbesondere den Peptidrest FEPQK und seine N-terminal verkürzten Fragmente und Z_{C26} einen Aminosäurerest oder einen Peptidrest von bis zu 30 Aminosäuren, insbesondere den Peptidrest ISIISHEY, bedeutet.

23. Peptid nach Anspruch 1 mit der Aminosäuresequenz
(xx)hBD-30
Z_{N27}-CFNKVTGYCRKKCKVGERYEIGCLSGKLCC-Z_{C27}
Z_{N27} einen Aminosäurerest oder einen Peptidrest von bis zu 30 Aminosäuren, insbesondere den Peptidrest LKK und seine N-terminal verkürzten Fragmente und Z_{C27} einen Aminosäurerest oder einen Peptidrest von bis zu 30 Aminosäuren, insbesondere den Peptidrest ANDEEEK, bedeutet.

24. Peptid nach Anspruch 1 mit der Aminosäuresequenz
(yy)hBD-31
Z_{N28}-CLNDVGICKKKCKPEEMHVKNGWAMCGKQRDCC-Z_{C28}
Z_{N28} einen Aminosäurerest oder einen Peptidrest von bis zu 30 Aminosäuren, insbesondere den Peptidrest WYVKK und seine N-terminal verkürzten Fragmente und Z_{C28} einen Aminosäurerest oder einen Peptidrest von bis zu 30 Aminosäuren, insbesondere den Peptidrest VPADR, bedeutet.

25. Peptid nach Anspruch 1 mit der Aminosäuresequenz
(zz) hBD-32
Z_{N29}-CWNFRGSCRDECLKNERVYVFCVSGKLCC-Z_{C29}
Z_{N29} einen Aminosäurerest oder einen Peptidrest von bis zu 30 Aminosäuren, insbesondere den Peptidrest IET und seine N-terminal verkürzten Fragmente und Z_{C29} einen Aminosäurerest oder einen Peptidrest von bis zu 30 Aminosäuren, insbesondere den Peptidrest LK, bedeutet.

26. Peptide nach einem der Ansprüche 1 bis 25, wobei die Peptide die zyklischen, amidierten, acetylierten, sulfatierten, phosphorylierten, glycosylierten, und oxydierten Derivate sowie Peptidfragmente, die aus den oben beschriebenen Aminosäuresequenzen abgeleitet werden und eine ähnliche biologische Aktivität aufweisen, sind.

27. Arzneimittel, enthaltend eines oder mehrerer der Defensinpeptide oder seiner Derivate oder seiner Fragmente nach mindestens einem der Ansprüche 1 bis 26 als aktiven Wirkstoff neben üblichen Hilfs- und Zusatzstoffen.

28. Verwendung eines oder mehrerer der Defensinpeptide oder seiner Derivate oder seiner Fragmente nach mindestens einem der Ansprüche 1 bis 26 zur Behandlung von Erkrankungen, die bei bakteriellen Organbesiedlungen entstehen.

29. Verwendung eines oder mehrerer der Defensinpeptide oder seiner Derivate oder seiner Fragmente nach mindestens einem der Ansprüche 1 bis 26 zur Behandlung von Erkrankungen des menschlichen Organismus, insbesondere mit Beteiligung des Magen-Darm-Traktes, der Atemwege und des Urogenitalapparates.

30. Verwendung eines oder mehrerer der Defensinpeptide oder seiner Derivate oder seiner Fragmente nach mindestens einem der Ansprüche 1 bis 26 zur Behandlung der Störung der Fertilität, insbesondere bei Krankheiten der mit Oocyten verbundenen Spermienpenetrationsstörungen und Inidationsstörungen sowie Maturationsstörungen im männlichen Reproduktionsapparates, sowie als Kontrazeptivum.

31. Verwendung eines oder mehrerer der Defensinpeptide oder seiner Derivate oder seiner Fragmente nach mindestens einem der Ansprüche 1 bis 26 in verschiedenen galenischen Applikationsformen, insbesondere der lyophilisierten, mit Mannit aufgenommenen Form in sterilen Ampullen zur Auflösung in physiologischer Kochsalzlösung und/oder Infusionslösungen zur wiederholten Einzelinjektion und/oder Dauerinfusion in Mengen von 300 Mikrogramm bis 300 Milligramm eines oder mehrerer der Defensinpeptide nach Anspruch 1 pro Therapie-Einheit.

32. Verwendung der von den Defensinpeptiden nach mindestens einem der Ansprüche 1 bis 26 abgeleiteten Gensonden und Genen zur lokalen und systemischen Gentherapie der Indikationen gemäß einem der Ansprüche 28 bis 30 in epithelialen Geweben und Organen.
